# EUROPEAN PATENT APPLICATION

(11) **EP 2 166 355 A1**
(43) Date of publication of application: **24.03.2010**
(21) Application number: 08164535.0
(22) Date of filing: 17.09.2008
(51) Int. Cl.: G01N 33/50, G01N 33/497

(54) **Method for the diagnosis of chronic obstructive pulmonary disease by detecting volatile organic compounds in exhaled air**

(71) Applicant: Universiteit Maastricht, 6211 LK Maastricht (NL); Academisch Ziekenhuis Maastricht, 6229 HX Maastricht (NL)
(72) Inventor: van Berkel, Joep Joseph Benjamin Nathan, 6224 CS Maastricht (NL); Dallinga, Jan Willem, 6411 VA Heerlen (NL); Wouters, Emiel Frans, 2440 Geel (BE); Godschalk, Roger Wilhelmus Laurentius, 6369 TK Simpelveld (NL); van Schooten, Frederik Jan, 6247 BB Gronsveld (NL)
(74) Representative: Habets, Winand

(57) **Abstract**

The present invention relates to the identification of markers for the disease conditions related to Chronic Obstructive Pulmonary Disease (COPD). We describe the identification of a set of volatile organic compound (VOC) markers that can be used in the diagnosis of COPD. More in particular we found that these markers can be used in the early diagnosis of COPD. Moreover, these markers are useful in the prediction of the occurrence of COPD even when no other clinical signs are apparent yet. The method is useful since it provides a new non-invasive, easy, safe, cost effective and fast diagnostic tool for the diagnosis of COPD.

The invention therefore relates to a method for the diagnosis of COPD by detecting the presence of at least six VOCs in exhaled air wherein said at least 6 VOCs are isoprene, C16 hydrocarbon, 4,7-dimethyl undecane, 2,6-dimethyl heptanes, 4-methyl octane and hexadecane.

## Description

### Field of the invention

The present invention relates to the identification of markers for the disease conditions related to Chronic Obstructive Pulmonary Disease (COPD). The uses of such markers in diagnosis and a novel method for their identification are herein described.

### Background of the invention

Chronic obstructive pulmonary disease (COPD) is currently defined as a chronic disease showing irreversible obstruction of airways resulting from the progression of two major underlying diseases including chronic bronchitis and pulmonary emphysema. Chronic bronchitis is defined clinically as the persistence of cough, sputum, and difficult breathing, and pulmonary emphysema is defined histopathologically as an irreversible destruction of airway walls distal to the terminal bronchiole and clinically shows slowly progressive respiratory difficulties (GOLD workshop summary, Am J Respir Crit Care Med 2001; 163: 1256-1276).

Current definition of COPD excludes a bronchial asthma showing reversible obstruction of airways. COPD is one of the leading causes of death in the United States and Europe, and causes of death in patients with COPD are complications of the disease such as respiratory failure or infection.

Epidemiological data from the national health interview survey and the third national health and nutrition and examination survey in the United States illustrate relationships in the prevalence of asthma and COPD in nationally representative samples. Asthma prevalence in adults declines in weight from 5 to 10% at age 20 to 40 to 4 to 8% above age 60. COPD is uncommon in adults under age 40 but steadily increases with age, surpassing asthma in older adults. These findings suggest that asthma screening is most useful in adults up to approximately age 40, after which COPD screening and differential diagnoses are of comparable or greater utility.

Currently, smoking is regarded as the most important risk factor for the development of COPD. And secretions of many inflammatory mediators (IL-8 etc.) from airway epithelial cells stimulated by smoking and other factors including air pollution or chronic infection by bacteria or virus are considered to be responsible for the development of chronic inflammation of airway tissue in COPD.

However, the reason for the chronic persistence of inflammation after acute inflammation induced by those external stimuli is not determined yet. Accordingly, it is suggested that other pathogenetic mechanisms besides smoking may be involved in the pathogenesis of COPD (O'Byrne PM, Postma DS. Am J Respir Crit Care Med 1999: 159, pages S41-S66).

Diagnosis and therapy of pulmonary diseases is based on published medical guidelines like for example of the American Thoracic Society, Standards for the Diagnosis and Care of Patients with Chronic Obstructive Pulmonary disease, Am J Respir. Crit. Care Med. Vol. 52, pages S77-S120, 1995, Siafakas, N. M. , Vermeire, P, Pride, N. B., ERS Consensus Statement: Optimal Assessment and Management of Chronic Obstructive Pulmonary Disease (COPD), Eur Respir J, 8,1398- 1420, 1995; National Heart, Lung and Blood Institute, 1995. Global Initiative for Asthma. U. S. Government Printing Office, Washington, DC. Publication No.(NIH) 95-3659.

Currently, there exist a number of diagnostic tools for determining whether a patient suffers from COPD. Branchoalveolar lavage and bronchial biopsies are relatively invasive methods with a moderate specificity. A major limitation for the use of Branchoalveolar lavage is the large range of normal values for each parameter which makes this method insensitive in detecting disease. Moreover, both methods cause great discomfort for the patient.

The induced sputum method (Eur. Resp. J. 2000; 16, 355-359) also causes great discomfort for a patient but has a high specificity and sensitivity. Methods causing discomfort for a patient are not preferred in the art.

The present invention addresses the need in the art to have reliable, fast, affordable and simple methods for the diagnosis of COPD that cause as little discomfort for the patient as possible.

The use of questionnaires (Respir. Med. 2005; 99, 1311-1318 ) is the least invasive method available, however, this method has limited sensitivity (less than 90%) and specificity (less than 80%).

Spirometry is a non-invasive method with a high sensitivity and specificity. Diagnosis of chronic obstructive pulmonary disease can be made when the patients complain of typical clinical symptoms (cough, sputum, and dyspnea, etc.) and their FEV1 (forced expiraratory volume in one second) measured following the inhalation of bronchodilator showed less than 80% of predicted value and the ratio of FEV1/FVC was less than 70% on pulmonary function test (FVC = forced vital capacity).

A system for assisting in the diagnosis of functional lung diseases based on spirometric measurement data is known from EP 1271384A1. However, in order to be conclusive, the analysis has to be repeated over several periods of 6 months making this method rather cumbersome and time consuming.

WO2006118522 describes a method for the diagnosis of COPD using peptide markers within the biological fluids of patients.

Consequently, there is a need in the art to identify a reliable and straightforward indicator of the COPD disease state both in order to reliably distinguish the symptoms of COPD from those of other respiratory diseases and to predict changes in disease severity and progression, and response to medicine, even before these changes are manifest clinically.

The present invention provides a set of markers that may be measured in the exhaled air of a patient in order to reliably (i.e. with high specificity and sensitivity) predict that the patient is or will be suffering from COPD.

Biochemical and molecular biological markers are commonly accepted as valuable diagnostic tools in medicine and toxicology. A marker can be described as "a characteristic that is objectively measured and evaluated as an indicator of biologic processes, pathogenic processes, or pharmacological responses to a therapeutic intervention". A marker is any identifiable and measurable indicator associated with a particular condition or disease or a predisposition for a particular condition or disease.

Markers may have a predictive power, and as such may be used to predict or detect the presence, level, type or stage of particular conditions or diseases (including the presence or level of particular microorganisms or toxins), the susceptibility (including genetic susceptibility) to particular conditions or diseases, or the response to particular treatments (including drug treatments). It is thought that markers will play an increasingly important role in the future of drug discovery and development, by improving the efficiency of research and development programs. Markers can be used as diagnostic agents, monitors of disease progression, and monitors of treatment and predictors of clinical outcome. For example, various marker research projects are attempting to identify markers of specific cancers and of specific cardiovascular and immunological diseases.

There may or may not be a causal correlation between the presence or level of the marker and some aspect of the condition or disease (including the presence of, the level or changing level of, the type of, the stage of, the susceptibility to the condition or disease, or the responsiveness to a drug used for treating the condition or disease). The correlation may be qualitative, quantitative, or both qualitative and quantitative.

Typically a marker is a compound, compound fragment or group of compounds. Such compounds may be any compounds found in or produced by an organism, including proteins (and peptides), nucleic acids and other compounds. In particular, a marker as identified herein is a volatile organic compound (VOC).

Exhaled volatile compounds have been studied regarding their hypothesized function as biomarkers of oxidative stress as for example ethane. In 1971 Pauling et al. already demonstrated the availability of hundreds of different VOCs in exhaled air (Pauling, L., A. B. et al., 1971. Proc Natl Acad Sci U S A 68(10):2374-6) and many more have been identified since.

Ethane belongs to the group of volatile organic compounds (VOCs) and is demonstrated to be elevated in exhaled air of COPD patients compared to controls. A correlation was also demonstrated between levels of ethane and the degree of airway obstruction, smoking habits and FEV1 ( Paredi, P., S. A. et al., 2000. Am J Respir Crit Care Med 162(2 Pt 1):369-73.2).

In 2006, Barker et al. concluded that VOC analysis is feasible in exhaled air and holds potential for non-invasive diagnosis as demonstrated in young patients suffering from cystic fibrosis ( Barker, M., M. et al., 2006. Eur Respir J 27(5):929-36.).

Nitric oxide (NO) levels are generally accepted as an indication of inflammation and oxidative stress in the respiratory tract in for instance asthma (Kharitonov, S. A., and P. J. Barnes, 2001 Curr Opin Allergy Clin Immunol 1(3):217-24.). However, in COPD the use of NO is limited since exhaled NO levels are not or only marginally elevated in COPD patients (Kharitonov, S. A., and P. J. Barnes. 2001. Am J Respir Crit Care Med 163(7):1693-722.). There appears to be a negative correlation between exhaled NO levels and FEV1, the forced expiratory volume in one second (Tzortzaki, E. G., et al., Curr Med Chem 14(9):1037-48). Brindicci et al. observed slightly elevated alveolar NO and increased levels of exhaled NO during exacerbations in COPD patients (Brindicci, C., K. et al., 2005. Eur Respir J 26(1):52-9 and Bhowmik, A., T. A. et al., 2005. Eur Respir J 26(6):1009-15.).

Carbon monoxide (CO) has also been investigated as a COPD biomarker, and contrasting results are published. Yamaya et al. found a significant relationship between exhaled CO concentrations and FEV1, and exhaled CO appeared to correlate with the eosinophil count in sputum (Yamaya, M., K. et al., 1999. Eur Respir J 13(4):757-60) whereas others found no correlation of exhaled CO with lung function (Montuschi, P., S. A. et al., 2001. Chest 120(2):496-501). The application of CO as a diagnostic marker is also limited because exhaled CO levels are seriously affected by environmental CO, which may fluctuate considerably (Horvath, I., W. et al., 2001. summary of the ERS Research Seminar in Budapest, Hungary, September, 1999. Eur Respir J 18(2):420-30) and is influenced by active and passive smoking making its use as a biomarker for COPD at the least questionable.

Exhaled hydrogen peroxide (H2O2) has also been studied as a potential biomarker in exhaled breath since H2O2 levels are thought to reflect the underlying state of oxidative stress in the lungs. Schleiss et al. demonstrated that levels of exhaled H2O2 are higher in stable COPD patients compared to young non-diseased nonsmoking controls (Schleiss, M. B., O. et al., 2000. Eur Respir J 16(6):1115-8.). No difference in H2O2 levels was found in stable COPD patients compared to age and smoking status matched controls. Measuring exhaled H2O2 is not yet standardized and another disadvantage is the large intra-individual variability. Nonetheless one study has demonstrated exhaled H2O2 is further increased during exacerbations (Dekhuijzen, P. N., K. K. et al., 1996. Am J Respir Crit Care Med 154(3 Pt 1):813-6)

In conclusion, analysis of single compounds from exhaled air has proven to be hampered by its low sensitivity and specificity. In spite of all the different biomarkers that have been described for COPD, there is still a need in the art for more reliable markers, i.e. markers with a higher sensitivity and/or specificity for COPD.

### Summary of the invention

Chronic obstructive pulmonary disease (COPD) is an inflammatory condition characterized by oxidative stress. Volatile organic compounds (VOCs) are generated during this process and they are secreted via the lungs. We herein describe the identification of a set of VOC markers that can be used in the diagnosis of COPD. More in particular we found that these markers can be used in the early diagnosis of COPD. Moreover, these markers are useful in the prediction of the occurrence of COPD even when no other clinical signs are apparent yet. The method is useful since it provides a new non-invasive, easy, safe, cost effective and fast diagnostic tool for the diagnosis of COPD.

The invention therefore relates to an in vitro method for determining whether a patient suffers from COPD by detecting the presence or absence of at least six VOCs in exhaled air wherein said at least 6 VOCs are isoprene, C16 hydrocarbon, 4,7-dimethyl undecane, 2,6-dimethyl heptanes, 4-methyl octane and hexadecane and correlating the presence or absence of said at least 6 VOCs with the diagnosis of COPD.

### Detailed Description of the invention

The invention relates to a method for the in vitro diagnosis of COPD by detecting the level of at least six VOCs in exhaled air wherein said at least 6 VOCs are isoprene, C16 hydrocarbon, 4,7-dimethyl undecane, 2,6-dimethyl heptanes, 4-methyl octane and hexadecane.

Methods to measure VOCs in exhaled air are known in the art. Typically, exhaled air is collected e.g. in a bag and lead over an absorption medium capable of trapping VOCs. Trapped VOCs may be released and lead into a detector, such as a gas chromatography capillary column. VOCs may then be separated by gas chromatography and detected by a mass spectrometer equipped with a time-of-flight detector (TOF-MS). Analysis of the data output files from the GC-TOF-MS was performed in successive steps as previously described in detail (Van Berkel, J. J. et al., 2008. J Chromatogr B Analyt Technol Biomed Life Sci 861(1):101-7). Preferred ways of performing the method are further exemplified herein in the Examples section.

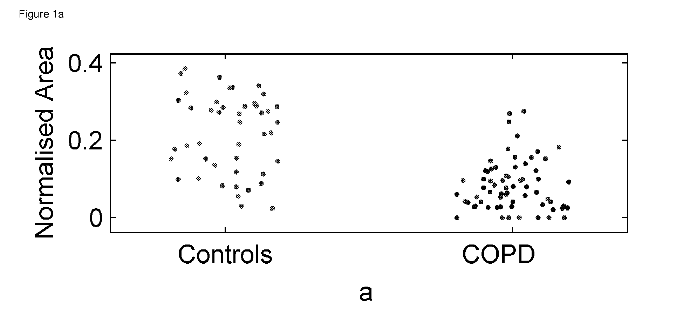

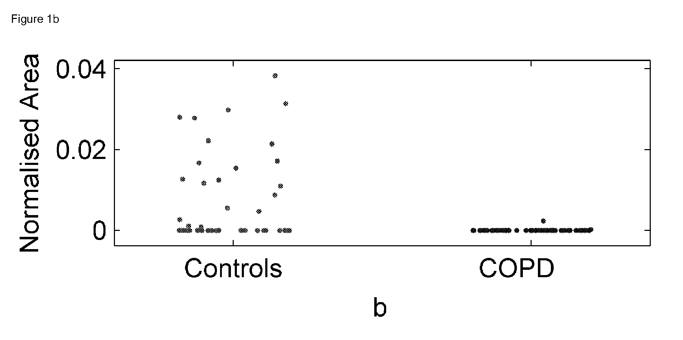

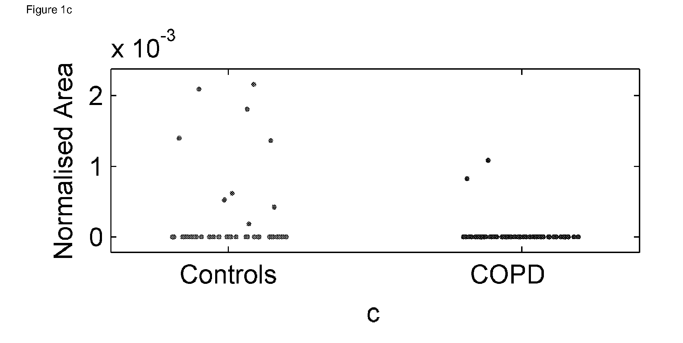

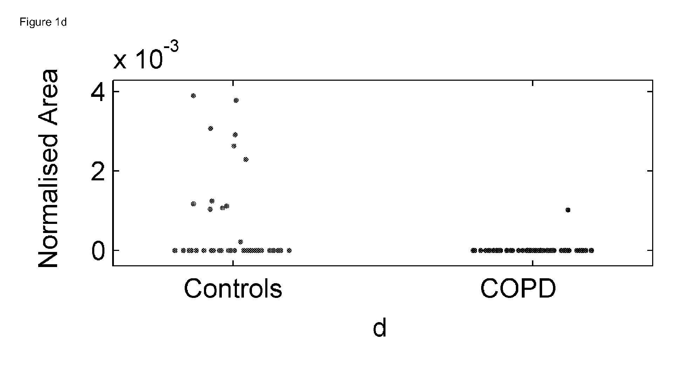

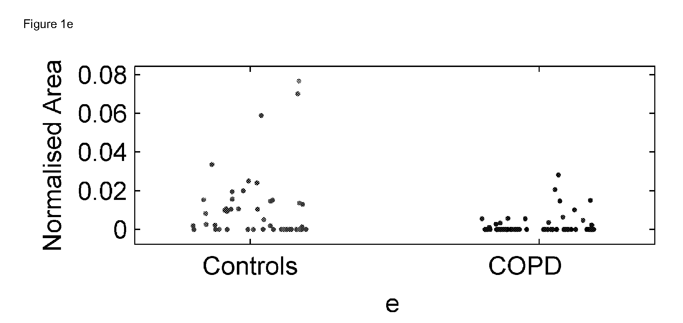

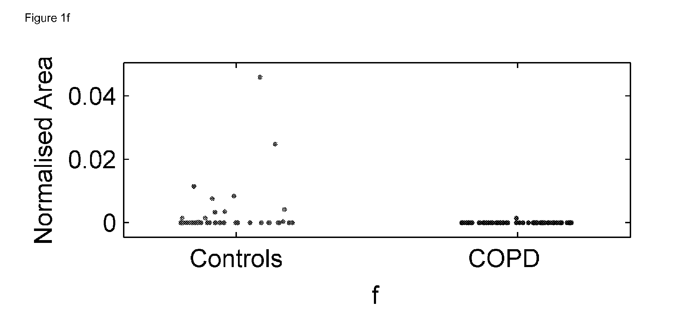

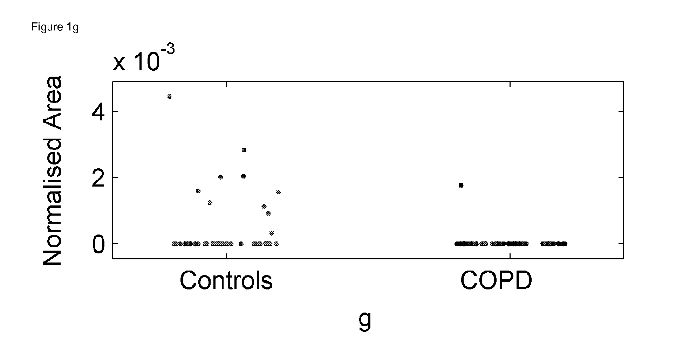

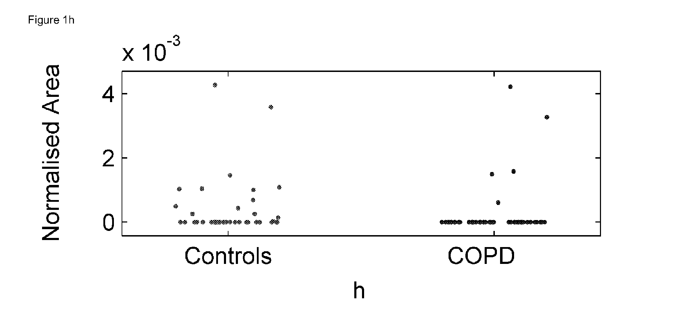

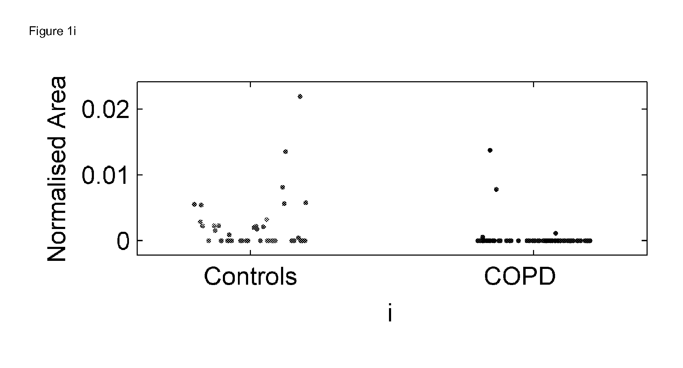

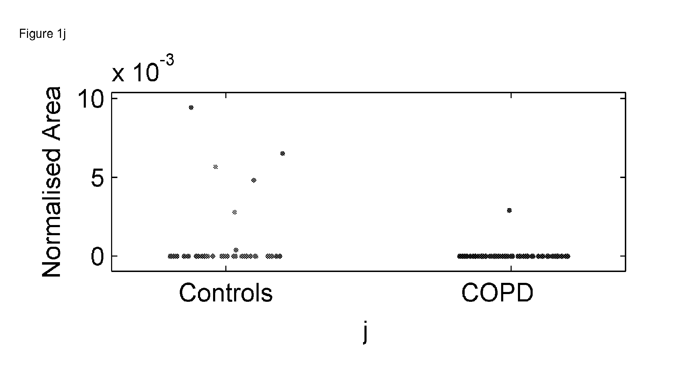

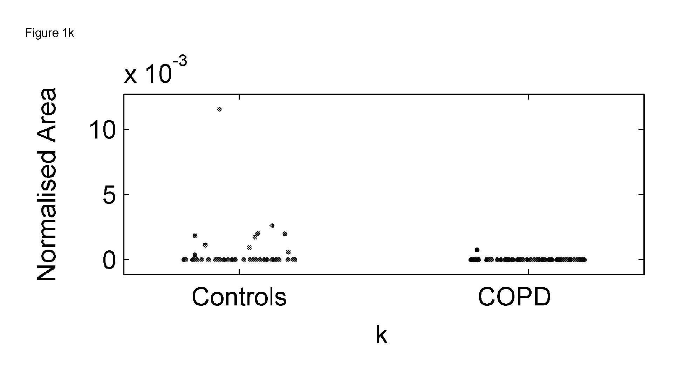

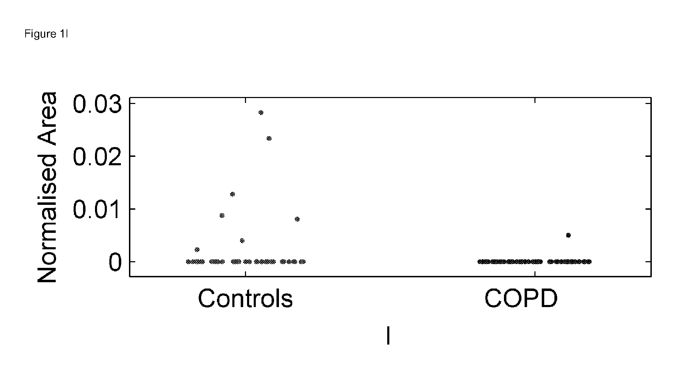

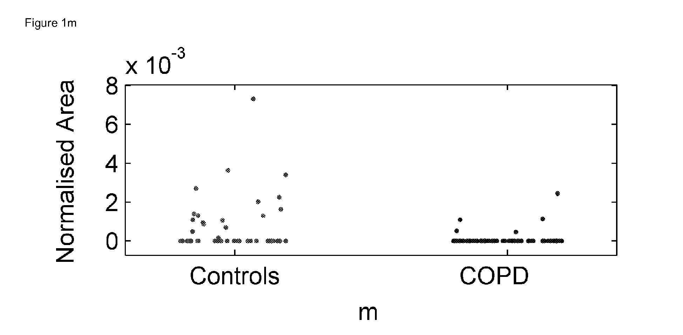

A method based on a profile of multiple VOCs markers proved to be more predictive and robust as compared to the analysis of a single marker available in exhaled air. We found that the level of a set of at least six markers is indicative for COPD. It was found that the levels of these six markers were remarkably lower than the levels in the control subjects. This is illustrated in figure 1.

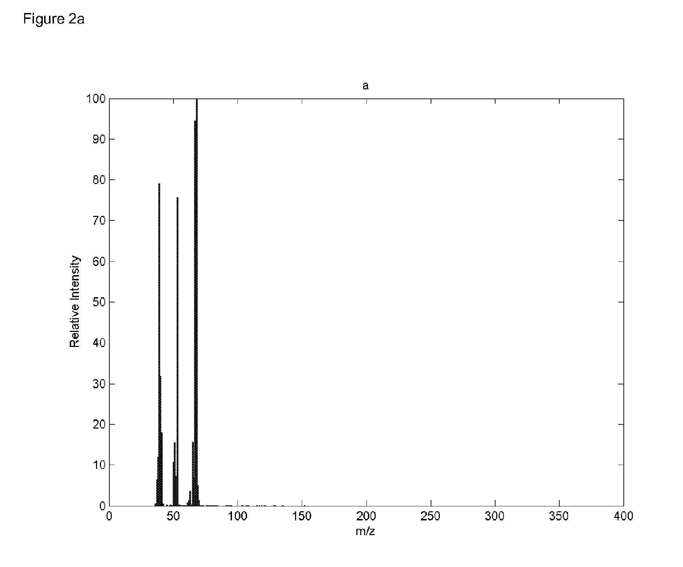

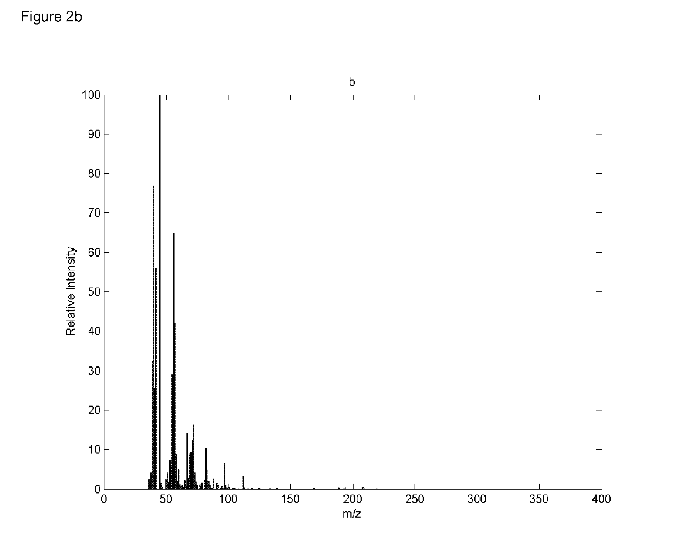

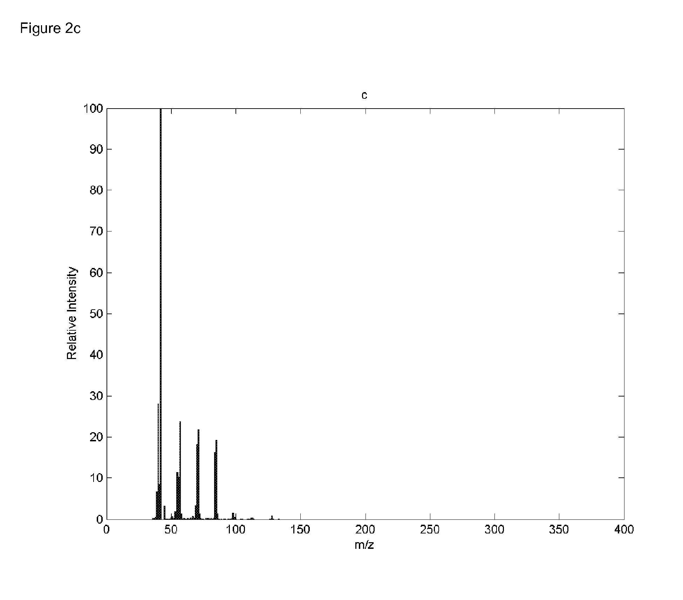

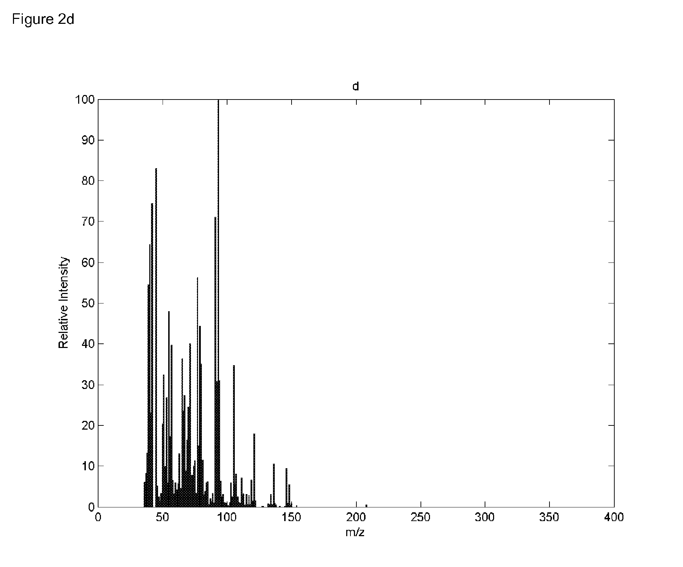

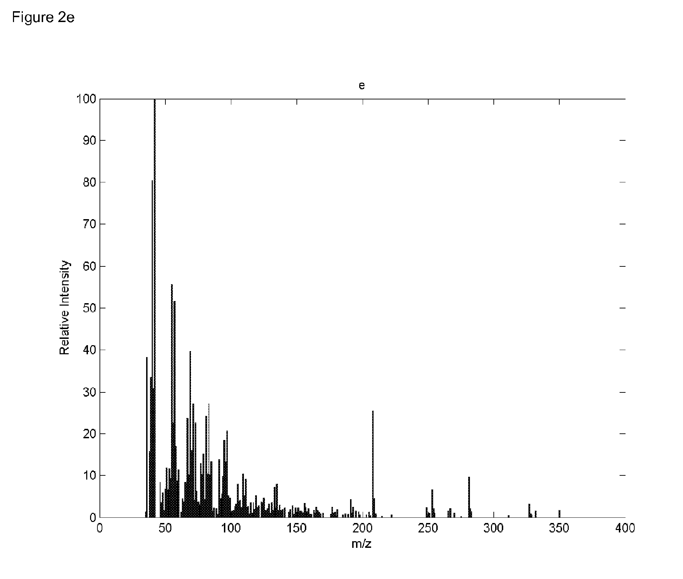

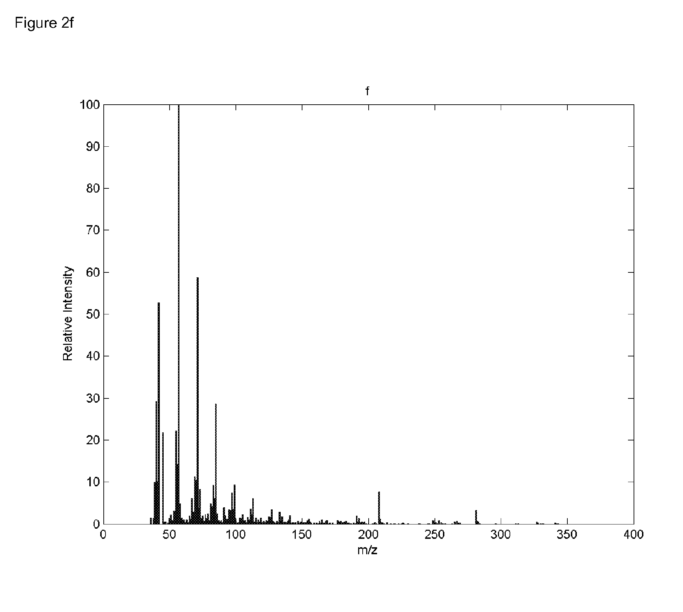

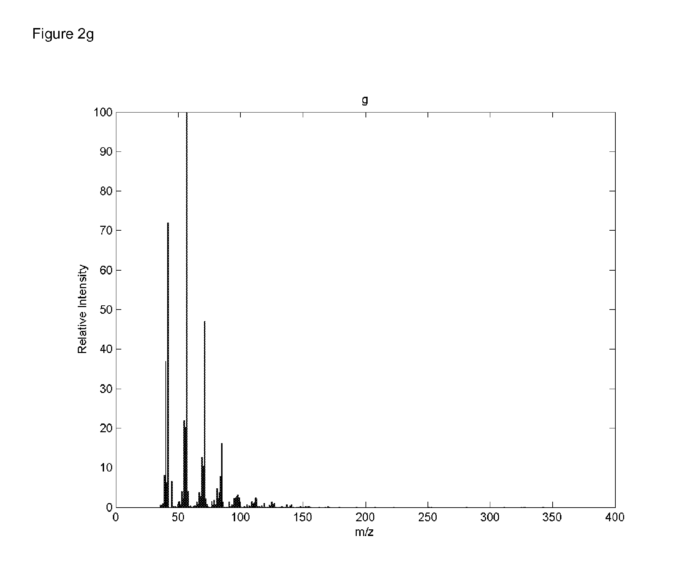

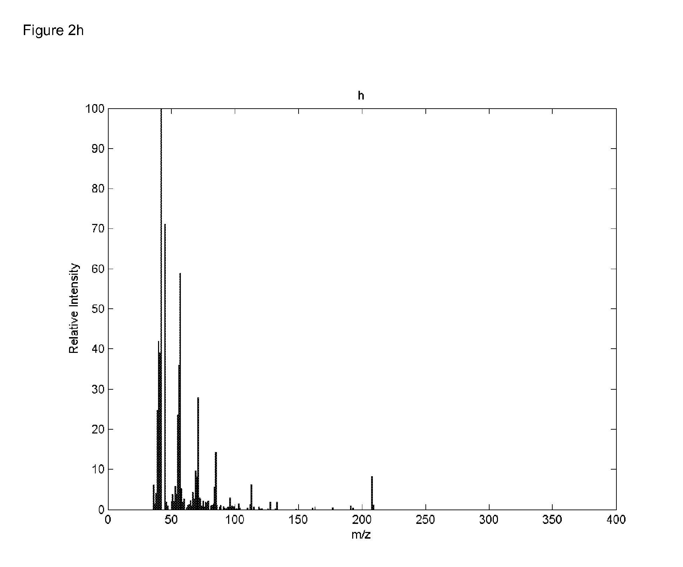

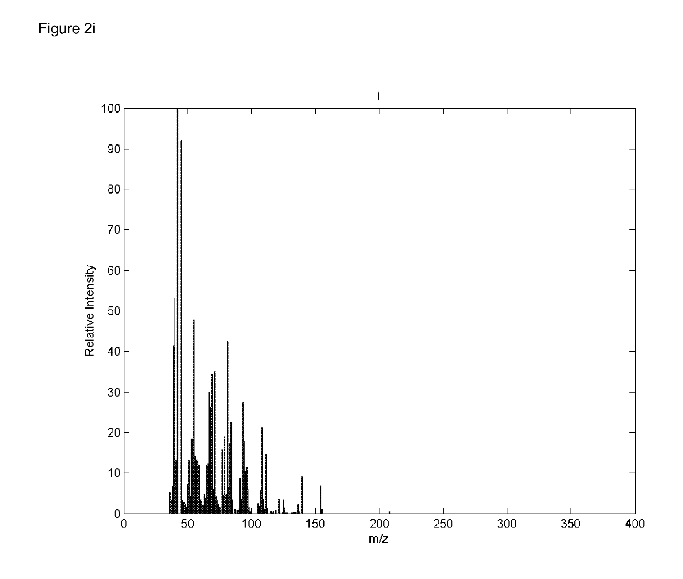

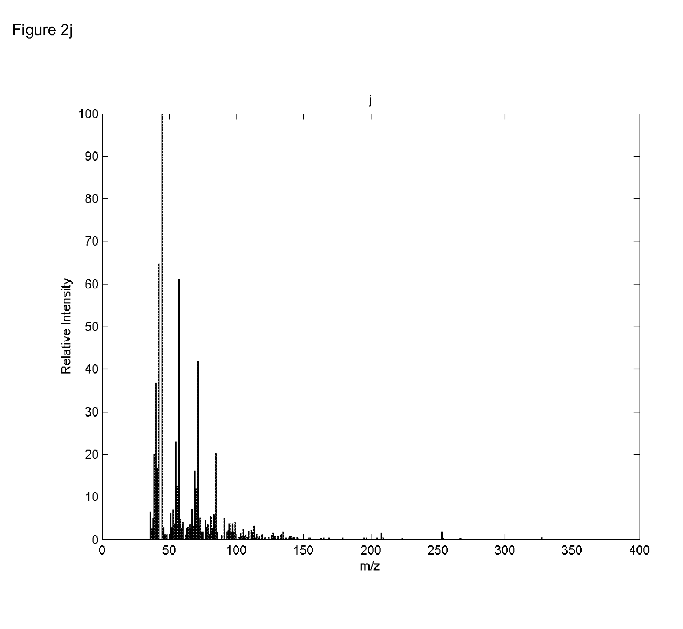

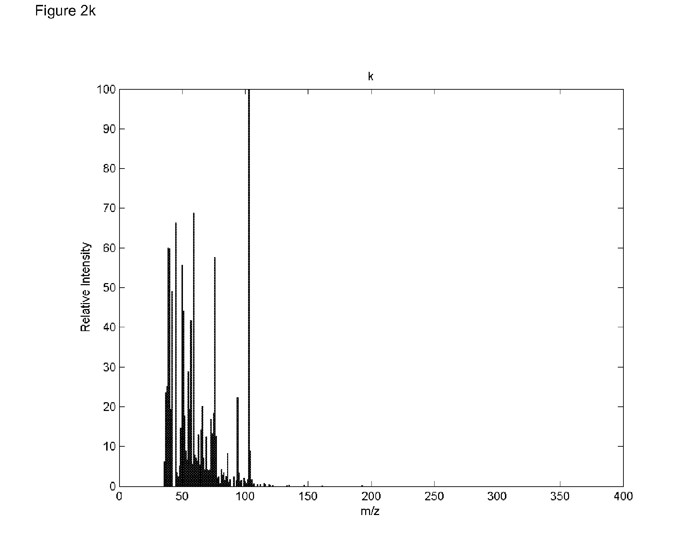

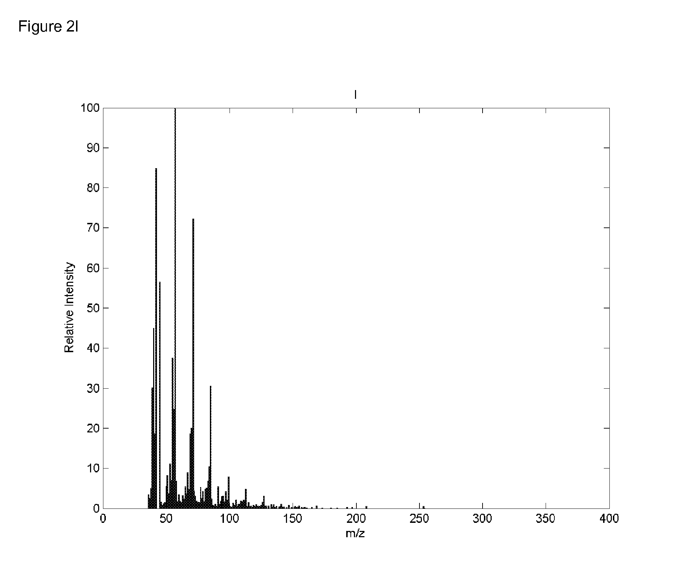

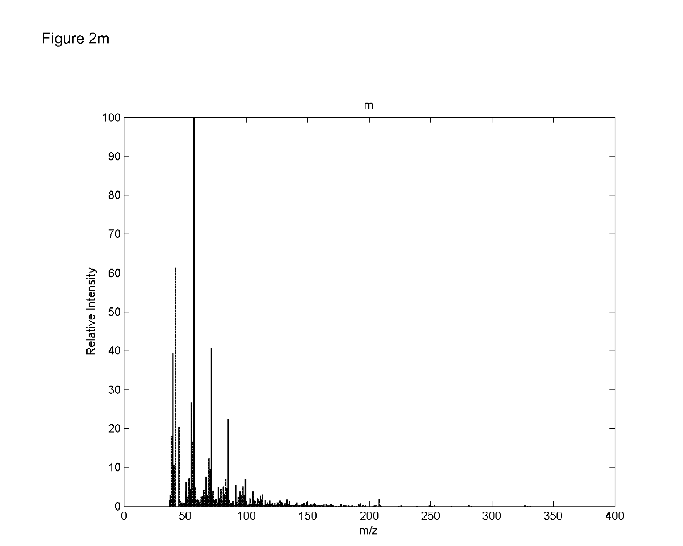

Each of the six markers identified herein is characterized by a typical TOF-MS spectrum. The spectrum of each component is shown in figure 2. Individual data underlying the spectra are presented in table 1 (retention times) and Table 2 (numerical data underlying the spectra of figure 2).

**Table 1 Compounds used in a method according to the invention.**

| | Chemical structure | RT (min.) |
|---|---|---|
| 1 | Isoprene | 2.6 |
| 2 | C16 hydrocarbon | 23.3 |
| 3 | 4,7-dimethyl undecane | 19.5 |
| 4 | 2,6- dimethyl heptane | 10.5 |
| 5 | 4-methyl octane | 11.3 |
| 6 | Hexadecane | 23.2 |

**Table 2: numerical data underlying the spectra of figure 2.**

| **isoprene** | | **C16 hydrocarbon** | | **4,7-dimethyl undecane** | | **2,6-dimethyl heptane** | | **4-methyl octane** | | **hexadecane** | |
|---|---|---|---|---|---|---|---|---|---|---|---|
| **m/z** | **counts** | **m/z** | **counts** | **m/z** | **counts** | **m/z** | **counts** | **m/z** | **counts** | **m/z** | **counts** |
| 36 | 1 | 36 | 2 | 36 | 6 | 36 | 1 | 36 | 3 | 37 | 2 |
| 37 | 6 | 37 | 2 | 37 | 8 | 38 | 1 | 37 | 2 | 38 | 3 |
| 38 | 12 | 38 | 4 | 38 | 13 | 39 | 10 | 38 | 5 | 39 | 18 |
| 39 | 79 | 39 | 32 | 39 | 54 | 41 | 29 | 39 | 30 | 41 | 40 |
| 41 | 32 | 41 | 77 | 41 | 64 | 42 | 10 | 41 | 45 | 42 | 11 |
| 42 | 18 | 42 | 26 | 42 | 23 | 43 | 53 | 42 | 19 | 43 | 61 |
| 43 | 1 | 43 | 56 | 43 | 74 | 45 | 22 | 43 | 85 | 45 | 20 |
| 50 | 11 | 45 | 100 | 45 | 83 | 46 | 1 | 45 | 57 | 46 | 1 |
| 51 | 16 | 46 | 1 | 46 | 5 | 50 | 1 | 46 | 2 | 47 | 1 |
| 52 | 7 | 50 | 3 | 47 | 3 | 51 | 2 | 47 | 1 | 48 | 1 |
| 53 | 76 | 51 | 4 | 48 | 1 | 52 | 1 | 48 | 1 | 49 | 1 |
| 61 | 1 | 52 | 2 | 49 | 3 | 53 | 3 | 49 | 1 | 50 | 4 |
| 62 | 1 | 53 | 7 | 50 | 20 | 54 | 3 | 50 | 5 | 51 | 6 |
| 63 | 4 | 54 | 6 | 51 | 32 | 55 | 22 | 51 | 8 | 52 | 2 |
| 65 | 16 | 55 | 29 | 52 | 10 | 56 | 14 | 52 | 4 | 53 | 7 |
| 66 | 7 | 56 | 65 | 53 | 27 | 57 | 100 | 53 | 11 | 54 | 4 |
| 67 | 95 | 57 | 42 | 54 | 6 | 58 | 5 | 54 | 7 | 55 | 27 |
| 68 | 100 | 58 | 9 | 55 | 48 | 59 | 1 | 55 | 38 | 56 | 17 |
| 69 | 5 | 59 | 2 | 56 | 17 | 60 | 1 | 56 | 25 | 57 | 100 |
| 70 | 1 | 60 | 5 | 57 | 40 | 61 | 1 | 57 | 100 | 58 | 5 |
| | | 61 | 1 | 58 | 6 | 63 | 1 | 58 | 7 | 59 | 2 |
| | | 62 | 1 | 59 | 3 | 65 | 2 | 59 | 2 | 60 | 2 |
| | | 63 | 1 | 60 | 6 | 66 | 1 | 60 | 3 | 61 | 2 |
| | | 65 | 2 | 61 | 4 | 67 | 6 | 61 | 2 | 62 | 1 |
| | | 66 | 1 | 62 | 6 | 68 | 3 | 62 | 1 | 63 | 2 |
| | | 67 | 14 | 63 | 13 | 69 | 11 | 63 | 3 | 64 | 3 |
| | | 68 | 3 | 64 | 5 | 70 | 11 | 64 | 2 | 65 | 4 |
| | | 69 | 9 | 65 | 36 | 71 | 59 | 65 | 5 | 66 | 2 |
| | | 70 | 9 | 66 | 24 | 72 | 4 | 66 | 4 | 67 | 8 |
| | | 71 | 12 | 67 | 27 | 73 | 8 | 67 | 9 | 68 | 3 |
| | | 72 | 16 | 68 | 9 | 74 | 1 | 68 | 5 | 69 | 12 |
| | | 73 | 4 | 69 | 16 | 75 | 2 | 69 | 19 | 70 | 9 |
| | | 74 | 2 | 70 | 24 | 76 | 1 | 70 | 20 | 71 | 41 |
| | | 75 | 1 | 71 | 40 | 77 | 2 | 71 | 72 | 72 | 3 |
| | | 77 | 1 | 72 | 5 | 78 | 1 | 72 | 4 | 73 | 4 |
| | | 78 | 1 | 73 | 8 | 79 | 2 | 73 | 3 | 74 | 2 |
| | | 79 | 2 | 74 | 10 | 80 | 1 | 74 | 2 | 75 | 2 |
| | | 81 | 2 | 75 | 11 | 81 | 5 | 75 | 2 | 76 | 1 |
| | | 82 | 10 | 76 | 3 | 82 | 4 | 76 | 1 | 77 | 5 |
| | | 83 | 5 | 77 | 56 | 83 | 9 | 77 | 5 | 78 | 2 |
| | | 84 | 2 | 78 | 15 | 84 | 6 | 78 | 3 | 79 | 4 |
| | | 85 | 1 | 79 | 44 | 85 | 29 | 79 | 4 | 80 | 1 |
| | | 88 | 3 | 80 | 35 | 86 | 2 | 80 | 2 | 81 | 5 |
| | | 91 | 1 | 81 | 12 | 87 | 1 | 81 | 5 | 82 | 3 |
| | | 92 | 1 | 82 | 3 | 88 | 1 | 82 | 5 | 83 | 7 |
| | | 95 | 1 | 83 | 4 | 89 | 1 | 83 | 7 | 84 | 5 |
| | | 97 | 6 | 84 | 6 | 91 | 4 | 84 | 10 | 85 | 22 |
| | | 98 | 1 | 85 | 6 | 92 | 2 | 85 | 30 | 86 | 2 |
| | | 112 | 3 | 86 | 1 | 93 | 1 | 86 | 2 | 87 | 1 |
| | | | | 87 | 2 | 94 | 1 | 87 | 1 | 88 | 1 |
| | | | | 88 | 1 | 95 | 3 | 88 | 1 | 89 | 1 |
| | | | | 89 | 3 | 96 | 3 | 89 | 1 | 91 | 5 |
| | | | | 90 | 1 | 97 | 7 | 91 | 5 | 92 | 1 |
| | | | | 91 | 71 | 98 | 3 | 92 | 1 | 93 | 2 |
| | | | | 92 | 31 | 99 | 9 | 93 | 2 | 94 | 2 |
| | | | | 93 | 100 | 100 | 1 | 94 | 3 | 95 | 4 |
| | | | | 94 | 31 | 103 | 1 | 95 | 3 | 96 | 3 |
| | | | | 95 | 6 | 104 | 1 | 96 | 2 | 97 | 5 |
| | | | | 96 | 3 | 105 | 2 | 97 | 4 | 98 | 3 |
| | | | | 97 | 3 | 106 | 1 | 98 | 2 | 99 | 7 |
| | | | | 98 | 1 | 107 | 1 | 99 | 8 | 100 | 1 |
| | | | | 99 | 1 | 109 | 2 | 103 | 1 | 102 | 1 |
| | | | | 102 | 1 | 110 | 1 | 104 | 1 | 103 | 2 |
| | | | | 103 | 6 | 111 | 4 | 105 | 2 | 104 | 1 |
| | | | | 104 | 3 | 112 | 2 | 107 | 1 | 105 | 4 |
| | | | | 105 | 35 | 113 | 6 | 108 | 1 | 106 | 1 |
| | | | | 106 | 6 | 115 | 1 | 109 | 2 | 107 | 1 |
| | | | | 107 | 8 | 117 | 1 | 110 | 2 | 108 | 1 |
| | | | | 108 | 3 | 118 | 1 | 111 | 2 | 109 | 2 |
| | | | | 109 | 1 | 119 | 1 | 112 | 2 | 110 | 1 |
| | | | | 110 | 1 | 121 | 1 | 113 | 5 | 111 | 3 |
| | | | | 111 | 7 | 123 | 1 | 115 | 1 | 112 | 2 |
| | | | | 112 | 3 | 124 | 1 | 117 | 1 | 113 | 3 |
| | | | | 113 | 3 | 125 | 2 | 119 | 1 | 115 | 2 |
| | | | | 114 | 1 | 126 | 2 | 120 | 1 | 116 | 1 |
| | | | | 115 | 3 | 127 | 3 | 121 | 1 | 117 | 1 |
| | | | | 116 | 1 | 128 | 1 | 123 | 1 | 119 | 1 |
| | | | | 117 | 3 | 131 | 1 | 125 | 1 | 120 | 1 |
| | | | | 118 | 1 | 133 | 3 | 126 | 2 | 121 | 1 |
| | | | | 119 | 7 | 134 | 1 | 127 | 3 | 123 | 1 |
| | | | | 120 | 1 | 135 | 2 | 128 | 1 | 125 | 1 |
| | | | | 121 | 18 | 137 | 1 | 129 | 1 | 126 | 1 |
| | | | | 122 | 2 | 139 | 1 | 131 | 1 | 127 | 1 |
| | | | | 132 | 1 | 140 | 1 | 133 | 1 | 128 | 1 |
| | | | | 133 | 1 | 141 | 2 | 135 | 1 | 129 | 1 |
| | | | | 134 | 3 | 147 | 1 | 141 | 1 | 131 | 1 |
| | | | | 135 | 1 | 149 | 1 | 147 | 1 | 133 | 2 |
| | | | | 136 | 11 | 154 | 1 | 155 | 1 | 135 | 1 |
| | | | | 137 | 1 | 155 | 1 | 169 | 1 | 140 | 1 |
| | | | | 146 | 9 | 163 | 1 | 208 | 1 | 141 | 1 |
| | | | | 147 | 1 | 165 | 1 | | | 145 | 1 |
| | | | | 148 | 6 | 168 | 1 | | | 147 | 1 |
| | | | | 150 | 1 | 169 | 1 | | | 149 | 1 |
| | | | | | | 177 | 1 | | | 155 | 1 |
| | | | | | | 179 | 1 | | | 163 | 1 |
| | | | | | | 183 | 1 | | | 191 | 1 |
| | | | | | | 191 | 2 | | | 193 | 1 |
| | | | | | | 193 | 1 | | | 208 | 2 |
| | | | | | | 197 | 1 | | | | |
| | | | | | | 208 | 8 | | | | |
| | | | | | | 209 | 1 | | | | |
| | | | | | | 249 | 1 | | | | |
| | | | | | | 253 | 1 | | | | |
| | | | | | | 265 | 1 | | | | |
| | | | | | | 267 | 1 | | | | |
| | | | | | | 281 | 3 | | | | |
| | | | | | | 282 | 1 | | | | |

The method according to the invention may be performed by first determining the optimal cut-off value for each individual marker. This may somewhat depend on the system and methods used for the detection of the VOCs. The skilled person will be aware of the mathematical principles underlying the determination of optimal cut-off values.

Exemplified herein is the calibration for a detection method using GC-TOF-MS wherein a training group of 50 COPD patients and 29 non-diseased controls are used. Patients and controls exhaled in a bag and the level of the six VOC markers (isoprene, C16 hydrocarbon, 4,7-dimethyl undecane, 2,6-dimethyl heptanes, 4-methyl octane and hexadecane) was determined using GC-TOF-MS. Figure 1 shows the relative amount of the component detected in the exhaled air from subjects in the training population.

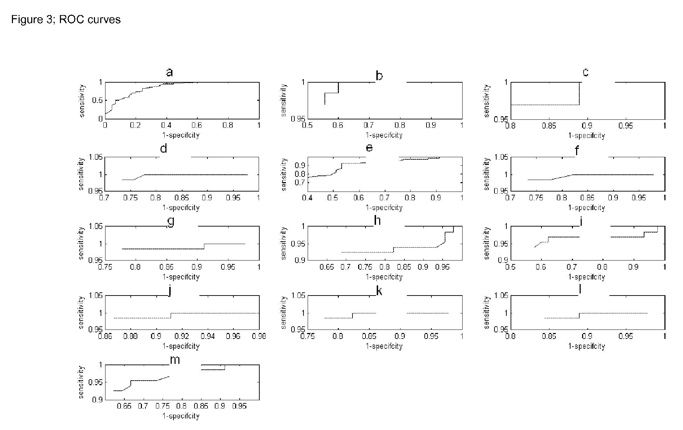

Based on these data, the optimal cut-off value was determined for each marker. For that purpose, the maximum value obtained with that particular marker was divided by 100, and the sensitivity and specificity was determined for an assay using this particular value as the cut-off. These calculations were repeated 100 times in incremental steps of highest value/100. From these 100 cut-off values, an optimal value was established. The individual ROC curves for the markers disclosed herein are shown in Figure 3.

Next, the optimal cut-off values thus obtained were used to validate the performance of the markers. A number of 16 COPD patients and 16 normal healthy controls exhaled in a bag and the VOC in the exhaled air was tested as described above for the training group and detailed in the Examples section. The method based on the 6 VOC markers (isoprene, C16 hydrocarbon, 4,7-dimethyl undecane, 2,6-dimethyl heptanes, 4-methyl octane and hexadecane) performed very well and identified 29 out of the 32 subjects correctly (91 % accuracy). Sensitivity was found to be 100% and specificity 81%.

In order to be sure that the 6 VOCs were not discriminating between controls and COPD patients because of the use of medication by COPD patients, their performance was tested on exhaled air samples obtained from 15 steroid naïve COPD patients. Fourteen out of these 15 subjects suffering from COPD not using any medication were correctly classified as COPD by the method according to the invention, demonstrating a sensitivity in this cohort of 94%.

If the sensitivity or specificity of the method is to be increased, further VOC markers may be added to the set of six markers as described above. For such an improved assay, at least one VOC marker selected from the group consisting of 3,7-dimethyl 1,3,6-octatriene,2,4,6-trimethyl decane, hexanal, benzonitrile, octadecane, undecane and terpineol may be added to the group of the 6 VOC markers as described above. The retention times of these markers is shown in Table 3. Table 4 shows the numerical data underlying the spectra of figure 2. The individual performance of these markers is shown in Table 5.

**Table 3 Compounds used in a method according to the invention.**

| | Chemical structure | RT (min.) |
|---|---|---|
| 7 | 3,7-dimethyl 1,3,6-octatriene | 14.5 |
| 8 | 2,4,6-trimethyl decane | 19.5 |
| 9 | hexanal | 9.9 |
| 10 | benzonitrile | 14.1 |
| 11 | octadecane | 25.5 |
| 12 | undecane | 16 |
| 13 | terpineol | 14.9 |

**Table 4; numerical data underlying the spectra of figure 2.**

| **3,7-dimethyl 1,3,6-octatriene** | | **2,4,6-trimethyl decane** | | **hexanal** | | **benzonitrile** | | **octadecane** | | **undecane** | | **terpineol** | |
|---|---|---|---|---|---|---|---|---|---|---|---|---|---|
| **m/z** | **counts** | **m/z** | **counts** | **m/z** | **counts** | **m/z** | **counts** | **m/z** | **counts** | **m/z** | **counts** | **m/z** | **counts** |
| 36 | 6 | 39 | 7 | 38 | 1 | 36 | 5 | 36 | 3 | 36 | 7 | 36 | 6 |
| 37 | 1 | 41 | 28 | 39 | 8 | 37 | 3 | 38 | 1 | 37 | 2 | 37 | 24 |
| 38 | 4 | 42 | 9 | 41 | 37 | 38 | 7 | 39 | 3 | 38 | 5 | 38 | 25 |
| 39 | 25 | 43 | 100 | 42 | 6 | 39 | 42 | 41 | 7 | 39 | 20 | 39 | 60 |
| 41 | 42 | 45 | 3 | 43 | 72 | 41 | 53 | 42 | 3 | 41 | 37 | 41 | 60 |
| 42 | 39 | 51 | 1 | 45 | 7 | 42 | 13 | 43 | 9 | 42 | 17 | 42 | 19 |
| 43 | 100 | 53 | 2 | 50 | 1 | 43 | 100 | 45 | 100 | 43 | 65 | 43 | 49 |
| 45 | 71 | 54 | 1 | 51 | 1 | 45 | 92 | 46 | 1 | 45 | 100 | 45 | 66 |
| 46 | 2 | 55 | 11 | 52 | 1 | 46 | 3 | 48 | 1 | 46 | 3 | 46 | 4 |
| 47 | 1 | 56 | 10 | 53 | 4 | 47 | 3 | 50 | 1 | 47 | 1 | 47 | 2 |
| 50 | 2 | 57 | 24 | 54 | 2 | 48 | 2 | 51 | 1 | 48 | 1 | 48 | 5 |
| 51 | 4 | 58 | 1 | 55 | 22 | 49 | 2 | 52 | 1 | 51 | 6 | 49 | 15 |
| 52 | 2 | 67 | 1 | 56 | 20 | 50 | 7 | 53 | 1 | 52 | 3 | 50 | 56 |
| 53 | 6 | 69 | 3 | 57 | 100 | 51 | 13 | 54 | 1 | 53 | 7 | 51 | 44 |
| 54 | 4 | 70 | 18 | 58 | 4 | 52 | 4 | 55 | 5 | 54 | 4 | 52 | 18 |
| 55 | 24 | 71 | 22 | 65 | 1 | 53 | 18 | 56 | 2 | 55 | 23 | 53 | 9 |
| 56 | 36 | 72 | 1 | 66 | 1 | 54 | 10 | 57 | 5 | 56 | 12 | 54 | 7 |
| 57 | 59 | 84 | 16 | 67 | 4 | 55 | 48 | 58 | 2 | 57 | 61 | 55 | 29 |
| 58 | 5 | 85 | 19 | 68 | 3 | 56 | 14 | 59 | 1 | 58 | 5 | 56 | 19 |
| 59 | 2 | 86 | 1 | 69 | 13 | 57 | 13 | 60 | 1 | 59 | 3 | 57 | 42 |
| 60 | 3 | 98 | 2 | 70 | 10 | 58 | 13 | 65 | 1 | 60 | 4 | 58 | 6 |
| 62 | 1 | 128 | 1 | 71 | 47 | 59 | 12 | 66 | 1 | 62 | 1 | 59 | 69 |
| 63 | 1 | | | 72 | 2 | 60 | 3 | 67 | 2 | 63 | 3 | 60 | 8 |
| 64 | 1 | | | 73 | 1 | 61 | 3 | 68 | 1 | 64 | 3 | 61 | 7 |
| 65 | 2 | | | 77 | 1 | 62 | 2 | 69 | 4 | 65 | 3 | 62 | 6 |
| 66 | 1 | | | 79 | 2 | 63 | 5 | 70 | 1 | 66 | 3 | 63 | 13 |
| 67 | 4 | | | 80 | 1 | 64 | 4 | 71 | 2 | 67 | 7 | 64 | 5 |
| 68 | 3 | | | 81 | 5 | 65 | 12 | 73 | 2 | 68 | 3 | 65 | 14 |
| 69 | 10 | | | 82 | 2 | 66 | 12 | 74 | 1 | 69 | 16 | 66 | 20 |
| 70 | 8 | | | 83 | 4 | 67 | 30 | 77 | 1 | 70 | 12 | 67 | 7 |
| 71 | 28 | | | 84 | 8 | 68 | 26 | 78 | 1 | 71 | 42 | 68 | 4 |
| 72 | 3 | | | 85 | 16 | 69 | 34 | 79 | 1 | 72 | 3 | 69 | 12 |
| 73 | 3 | | | 86 | 1 | 70 | 6 | 81 | 2 | 73 | 5 | 70 | 4 |
| 74 | 1 | | | 91 | 1 | 71 | 35 | 82 | 1 | 74 | 2 | 71 | 4 |
| 75 | 2 | | | 93 | 1 | 72 | 4 | 83 | 2 | 75 | 2 | 72 | 4 |
| 76 | 1 | | | 95 | 2 | 73 | 3 | 84 | 1 | 77 | 5 | 73 | 17 |
| 77 | 2 | | | 96 | 2 | 74 | 2 | 85 | 1 | 78 | 3 | 74 | 13 |
| 78 | 2 | | | 97 | 3 | 75 | 2 | 91 | 1 | 79 | 3 | 75 | 18 |
| 79 | 2 | | | 98 | 3 | 77 | 16 | 93 | 1 | 80 | 1 | 76 | 58 |
| 81 | 1 | | | 99 | 2 | 78 | 5 | 94 | 1 | 81 | 5 | 77 | 13 |
| 82 | 1 | | | 105 | 1 | 79 | 19 | 95 | 2 | 82 | 3 | 78 | 2 |
| 83 | 1 | | | 109 | 1 | 80 | 5 | 96 | 1 | 83 | 6 | 79 | 2 |
| 84 | 6 | | | 110 | 1 | 81 | 43 | 97 | 2 | 84 | 6 | 80 | 1 |
| 85 | 14 | | | 111 | 1 | 82 | 7 | 105 | 1 | 85 | 20 | 81 | 4 |
| 86 | 1 | | | 112 | 2 | 83 | 17 | 109 | 1 | 86 | 2 | 82 | 3 |
| 88 | 1 | | | 113 | 2 | 84 | 22 | 111 | 1 | 89 | 1 | 83 | 3 |
| 89 | 1 | | | 119 | 1 | 85 | 4 | 133 | 1 | 91 | 5 | 84 | 2 |
| 91 | 1 | | | 123 | 1 | 87 | 1 | 135 | 1 | 93 | 2 | 85 | 3 |
| 94 | 1 | | | 125 | 1 | 89 | 1 | 208 | 2 | 94 | 2 | 86 | 8 |
| 95 | 1 | | | 126 | 1 | 90 | 1 | 253 | 1 | 95 | 4 | 87 | 1 |
| 96 | 3 | | | 127 | 1 | 91 | 9 | 281 | 1 | 96 | 2 | 88 | 2 |
| 97 | 1 | | | 137 | 1 | 92 | 4 | | | 97 | 4 | 91 | 2 |
| 98 | 1 | | | 141 | 1 | 93 | 27 | | | 98 | 2 | 93 | 1 |
| 99 | 1 | | | | | 94 | 18 | | | 99 | 4 | 94 | 22 |
| 103 | 1 | | | | | 95 | 10 | | | 102 | 1 | 95 | 3 |
| 112 | 1 | | | | | 96 | 11 | | | 103 | 1 | 96 | 1 |
| 113 | 6 | | | | | 97 | 6 | | | 104 | 1 | 97 | 2 |
| 115 | 1 | | | | | 98 | 2 | | | 105 | 2 | 99 | 2 |
| 119 | 1 | | | | | 99 | 1 | | | 106 | 1 | 100 | 1 |
| 128 | 2 | | | | | 105 | 3 | | | 107 | 1 | 101 | 1 |
| 133 | 2 | | | | | 106 | 2 | | | 108 | 1 | 102 | 2 |
| 163 | 1 | | | | | 107 | 6 | | | 109 | 2 | 103 | 100 |
| 191 | 1 | | | | | 108 | 21 | | | 111 | 2 | 104 | 9 |
| 208 | 8 | | | | | 109 | 4 | | | 112 | 2 | 105 | 2 |
| 209 | 1 | | | | | 110 | 1 | | | 113 | 3 | 107 | 1 |
| | | | | | | 111 | 15 | | | 115 | 1 | 110 | 1 |
| | | | | | | 112 | 1 | | | 117 | 1 | 115 | 1 |
| | | | | | | 115 | 1 | | | 119 | 1 | | |
| | | | | | | 117 | 1 | | | 121 | 1 | | |
| | | | | | | 119 | 1 | | | 124 | 1 | | |
| | | | | | | 121 | 4 | | | 126 | 1 | | |
| | | | | | | 125 | 3 | | | 127 | 2 | | |
| | | | | | | 126 | 1 | | | 128 | 1 | | |
| | | | | | | 136 | 2 | | | 129 | 1 | | |
| | | | | | | 139 | 9 | | | 131 | 1 | | |
| | | | | | | 154 | 7 | | | 133 | 1 | | |
| | | | | | | 155 | 1 | | | 135 | 2 | | |
| | | | | | | 208 | 1 | | | 139 | 1 | | |
| | | | | | | | | | | 140 | 1 | | |
| | | | | | | | | | | 141 | 1 | | |
| | | | | | | | | | | 142 | 1 | | |
| | | | | | | | | | | 143 | 1 | | |
| | | | | | | | | | | 145 | 1 | | |
| | | | | | | | | | | 169 | 1 | | |
| | | | | | | | | | | 208 | 2 | | |
| | | | | | | | | | | 253 | 2 | | |
| | | | | | | | | | | 327 | 1 | | |

**Table 5. Sensitivity and specificities obtained when using the method according to the invention with optimized cut-off values. Patient groups tested were a training group, Validation Group and a steroid naïve group as described in the specification**

| Chemical structure | Training Group | | Validation Group | | Steroid naive group | |
|---|---|---|---|---|---|---|
| | Sens. | Spec. | Sens. | Spec. | Sens. | Spec. |
| isoprene * | 0.96 | 0.41 | 1.00 | 0.56 | 0.97 | 0.47 |
| C16 hydrocarbon * | 0.96 | 0.55 | 1.00 | 0.75 | 0.97 | 0.62 |
| 4,7- dimethyl undecane * | 0.96 | 0.69 | 1.00 | 0.75 | 0.97 | 0.71 |
| 2,6- dimethyl heptane * | 0.98 | 0.69 | 1.00 | 0.50 | 0.98 | 0.62 |
| 4-methyl octane * | 0.98 | 0.76 | 1.00 | 0.69 | 0.98 | 0.73 |
| Hexadecane * | 0.98 | 0.83 | 1.00 | 0.82 | 0.98 | 0.82 |
| 3,7-dimethyl 1,3,6-octatriene | 1.00 | 0.83 | 1.00 | 0.56 | 1.00 | 0.73 |
| 2,4,6-trimethyl decane | 1.00 | 0.90 | 0.94 | 0.69 | 0.98 | 0.82 |
| hexanal | 1.00 | 0.90 | 0.94 | 0.69 | 0.98 | 0.82 |
| benzonitrile | 1.00 | 0.93 | 0.94 | 0.69 | 0.98 | 0.84 |
| octadecane | 1.00 | 0.90 | 0.94 | 0.69 | 0.98 | 0.82 |
| undecane | 1.00 | 0.90 | 1.00 | 0.63 | 1.00 | 0.80 |
| terpineol | 1.00 | 1.00 | 1.00 | 0.75 | 1.00 | 0.91 |

A method for the diagnosis of COPD by detecting the level of 13 VOCs in exhaled air wherein said VOCs are isoprene, C16 hydrocarbon, 4,7-dimethyl undecane, 2,6-dimethyl heptanes, 4-methyl octane, hexadecane, 3,7-dimethyl 1,3,6-octatriene,2,4,6-trimethyl decane, hexanal, benzonitrile, octadecane, undecane and terpineol yielded a perfect score, i.e. a sensitivity of 100% and a specificity of 100% in the training group.

The amounts in exhaled air of the VOCs that performed best in the method according to the invention are predominantly lower in COPD patients than in controls. Without wanting to be bound by theory, an explanation for this observation may be found in the complicated biological equilibrium of formation and removal of VOCs in the human body. The hypothesis is that the inflammation driven oxidative stress is responsible for oxidizing macromolecules, including polyunsaturated fatty acids that are abundantly present in membranes, leading to a spectrum of breakdown products excreted as VOCs. Thus, the relative composition of VOCs in exhaled breath of COPD patients can change as a result of the disease, and this change can be either an increase or a decrease of certain components

Apart from the oxidative stress hypothesis, an alternative reason may be that lungs are remodeled during COPD resulting in altered gas exchanges over the blood lung barrier. Further studies are necessary in clinical settings but also in inflammation models to explain the biochemical origin, the physiological meaning and exhalation kinetics of our selected VOCs. Nevertheless, even without this mechanistic knowledge, the components may already be of value to establish a diagnostic tool for clinical settings.

### Legends to the figures

Figure 1 shows the relative amount of the component available in the exhaled air from subjects in the training population. Subject numbers located on the right represent COPD patients; subjects on the left represent the control group. Each graph represents one compound and every dot represents one subjects. The height of every dot represents the relative amount the compound that was available in the exhaled air sample. a) isoprene, b) C16 hydrocarbon, c) 4,7-dimethyl undecane, d) 2,6-dimethyl heptane, e) 4-methyl ocatane, f) Hexadecane, g) 3,7-dimethyl 1,3,6-octatriene, h) 2,4,6-trimethyl decane, i) Hexanal, j) Benzonitrile, k) Octadecane, I) Undecane, m) Terpineol.

Figure 2 shows the MS spectra of the VOC compounds according to the present invention. a) isoprene, b) C16 hydrocarbon, c) 4,7-dimethyl undecane, d) 2,6- dimethyl heptane, e) 4-methyl ocatane, f) Hexadecane, g) 3,7-dimethyl 1,3,6-octatriene, h) 2,4,6-trimethyl decane, i) Hexanal, j) Benzonitrile, k) Octadecane, I) Undecane, m) Terpineol.

Figure 3 shows the individual ROC curves of the compounds according to the invention. a) isoprene, b) C16 hydrocarbon, c) 4,7-dimethyl undecane, d) 2,6- dimethyl heptane, e) 4-methyl ocatane, f) Hexadecane, g) 3,7-dimethyl 1,3,6-octatriene, h) 2,4,6-trimethyl decane, i) Hexanal, j) Benzonitrile, k) Octadecane, I) Undecane, m) Terpineol.

### Examples

### Example 1: Study subjects

As a training population a total of 50 COPD patients and 29 non-diseased controls both smoking and non-smoking were recruited at Maastricht University, The Netherlands. Subject characteristics are shown in table 6. Diagnosis of COPD was based on examination of pulmonary function according to international guidelines. Subjects were sampled at centrally ventilated treatment rooms located in the hospital. Non-diseased control subjects were staff members. A medical interview confirmed absence of respiratory disease in these subjects. Another set of 16 COPD patients and 16 non-diseased controls were obtained from the Centre for Integrated Rehabilitation Organ Failure (CIRO). This population was used as a validation population to validate the results that were obtained in the training population. Additionally, a set of 15 persons was sampled at the University Hospital Maastricht diagnosed as COPD patients, and exhaled air was obtained from these patients before therapeutical treatment with steroids was initiated. This population was used to further validate the performance of the selected VOCs in diagnosing the disease. All subjects gave their informed consent and the study protocol was approved by the medical ethics committee of the University of Maastricht.

### Example 2: Sample collection and Analysis

Subjects were asked to exhale into resistance free Tedlar bags (5L) and the content of the bag was transported under standardized conditions onto stainless steel two-bed sorption tubes, filled with carbograph 1TD/Carbopack X (Markes International, Llantrisant, Wales, UK) that trap VOCs. This sampling approach sampled a mixture of alveolar air and dead space air, which is about 150 ml during tidal breathing. Participants were asked to inhale, hold their breath for 5 seconds and subsequently fully expire. Most of the subjects were able to fully inflate the Tedlar bag in 2 to 4 expirations.

The sorption tubes were placed inside a thermal desorption unit (Marks Unity desorption unit, Markes International Limited, Llantrisant, Wales, UK) and subsequently heated to 270°C in order to release all VOCs onto the gas chromatography capillary column (RTX-5ms, 30 m x 0.25 mm 5% diphenyl, 95 % dimethylsiloxane capillary, film thickness 1 µm). The desorption unit is highly suitable for repeated, quantitative and reproducible measurements. VOCs are separated by GC

**Table 6 Study subject characteristics**

| Study population | Training set | | Validation set | | Steroid naive |
|---|---|---|---|---|---|
| | Controls (n=29) | COPD (n=50) | Controls (n=16) | COPD (n=16) | COPD (n=15) |
| Age, yr | 50 ± 9 | 73 ± 8 | 51 ± 6 | 63 ± 6 | 57 ± 8 |
| Sex, M/F | 14/15 | 38/12 | 8/8 | 11/5 | 8/7 |
| FEV1, % predicted | 90 ± 14 | 50 ± 15 | 92 ± 7 | 53 ± 13 | 74 ± 12 |
| RV, % predicted | 77 ± 19 | 176 ± 34 | 72 ± 13 | 154 ± 29 | 148 ± 13 |
| Smoking status, current/ex/non | 9/7/13 | 38/6/6 | 3/3/10 | 2/14/0 | 3/9/3 |
| pack-years | 18 ± 7 | 49 ± 12 | 12 ± 5 | 35 ± 6 | 29 ± 26 |

(ThermoFisher Scientific., Austin, Texas, USA) and subsequently detected by a time-of-flight mass spectrometer (TOF-MS) (Thermo Electron Tempus Plus time-of-flight mass spectrometer, ThermoFisher Scientific, Austin, Texas, USA).

The temperature of the gas chromatograph was programmed as follows: 40 °C during 5 min., then raised with 10 °C/min until the final maximum temperature of 270 °C, this temperature was maintained for 5 min. Electron ionization mode at 70 eV was used with a 5Hz scanning rate over a mass range of m/z 35-350 amu.

### Example 3: Data-acquisition & mining

Analysis of the data output files from the GC-TOF-MS was performed in successive steps as previously described in detail (Van Berkel, J. J. et al., 2008. J Chromatogr B Analyt Technol Biomed Life Sci 861(1):101-7). In summary: the first step was to perform peak detection and baseline corrections on all analysis output files. Normalization of the calculated peak areas was performed using an area scaling factor. The rescaling factor used in this study is based on the cumulative area under the detected peaks, since all chromatograms display rather similar profiles and this method of normalization is most robust.

Next, retention times (RT) of all subjects were corrected for chromatographic drifting and lined up. Applying this correction for retention times is very effective and easy to perform eliminating the use of an added internal standard, adding to the straight-forwardness and easy to perform routine of the presented methodology.

Finally, the output files were merged by combining corresponding compounds based on degree of similarity of the corresponding mass spectra - by determining the match factor values (MFs) - and similarity of RT. The degree of mass spectra similarity was calculated as described by Stein et al. (Stein, S. E. et al., 1994. Journal of the American Society for Mass Spectrometry 5(9):859-866). These match factors were only determined for compounds within a selectable RT-window.

## Claims

1. In vitro method for determining whether a patient suffers from COPD by detecting the presence or absence of at least six VOCs in exhaled air wherein said at least 6 VOCs are isoprene, C16 hydrocarbon, 4,7-dimethyl undecane, 2,6-dimethyl heptanes, 4-methyl octane and hexadecane and correlating the presence or absence of said at least 6 VOCs with the diagnosis of COPD.

2. Method according to claim 1 wherein at least one additional VOC is detected selected from the group consisting of 3,7-dimethyl 1,3,6-octatriene, 2,4,6-trimethyl decane, hexanal, benzonitrile, octadecane, undecane and terpineol.
